# FASCICULE DE BREVET EUROPEEN

(11) **EP 0 727 662 B1**
(45) Date de publication et mention de la délivrance du brevet: **20.08.1997**
(21) Numéro de dépôt: 96400181.2
(22) Date de dépôt: 25.01.1996
(51) Int. Cl.: G01N 33/50

(54) **Test de matières actives sur cheveux épilés**
Test aktiver Materialien auf epilierten Haaren
Testing of active materials on depilated hair

(30) Priorité: 17.02.1995 FR 9501880
(43) Date de publication de la demande: 21.08.1996
(73) Titulaire: L'OREAL, 75008 Paris (FR)
(72) Inventeur: Mahe, Yann, F-91390 Morsang sur Orge (FR); Billoni, Nelly, F-75020 Paris (FR); Michelet, Jean-François, F-94000 Creteil (FR)
(74) Mandataire: Tezier Herman, Béatrice

(56) Documents cités:
- EP-A- 0 434 319
- FASEB JOURNAL, vol. 6, no. 3, 1992 WASHINGTON DC USA, pages 911-913, XP 000565635 J.J. JIMENEZ ET AL. 'Interleukin 1 protects hair follicles from cytarabine (ARA-C)-induced toxicity in vivo and in vitro.'

## Description

L'invention a pour objet un procédé de test d'une substance, ladite substance étant éventuellement active dans le domaine capillaire.

Par domaine capillaire, on entend tout ce qui peut avoir trait au cheveu ou au poil d'un individu. Ainsi, par "substance éventuellement active dans le domaine capillaire", ou simplement par la suite dans le texte par "substance", on entend toute molécule ou ensemble de molécules présentant une activité potentielle dans le domaine du capillaire, et en particulier toute molécule ou ensemble de molécules ayant potentiellement une activité sur la coloration, la survie, le ralentissement, l'arrêt de la croissance, la chute ou encore la pousse exacerbée des follicules pileux.
La substance à tester peut être utilisée selon le procédé de l'invention soit sous sa forme moléculaire, soit sous la forme d'une composition contenant la molécule à tester.

A ce jour, l'art antérieur évoque deux méthodes principales pour le test d'une substance éventuellement active dans le domaine capillaire.
La première consiste à pratiquer les essais sur des sujets volontaires et à constater de manière plus ou moins rapide les effets de la substance testée. Il est évident que cette méthode présente de nombreux désavantages dont en particulier celui de s'adresser à des êtres humains, ce qui à l'évidence limite pour des raisons d'éthique le champ d'application de la méthode. Ainsi le nombre et la qualité des substances testées se trouvent limités. De plus, ces essais sont en général lourds de mise en oeuvre et s'étalent sur des périodes de temps longues. Les résultats du test sont alors dans la plupart des cas des constatations de modifications phénotypiques du follicule pileux.

La deuxième méthode connue dans l'art antérieur est celle décrite dans le brevet EP 434 319. Cette méthode consiste à pratiquer les essais suivant un procédé qui fait intervenir quatre étapes dont en particulier une étape d'isolement d'un follicule pileux viable ayant conservé un bulbe intact.
Pour cela il s'avère nécessaire de pratiquer une microdissection du follicule pileux, à partir d'un prélèvement effectué sur un sujet. Celui-ci conserve de toute manière une trace de cette intervention. Cela limite l'approvisionnement en follicule pileux et la mise en oeuvre de la méthode de test est lourde et longue.

On trouve une application de la technique décrite dans le brevet EP 434 319 dans la publication de Jimenez J. J. et collaborateurs (FASEB Journal, 1992, vol.6, n°3, pages 911-913) dans laquelle il est fait état de résultats de l'étude de l'effet protecteur de l'interleukine 1 sur le follicule pileux mis en présence de cytarabine (ARA-C). Les expériences menées *in vitro* ont été réalisées avec des follicules pileux microdisséqués selon la méthode décrite dans EP 434 319.

Après de nombreux travaux, la demanderesse a pu montrer qu'il est possible d'utiliser dans le domaine du capillaire au moins un follicule pileux épilé pour tester une substance éventuellement active dans le domaine capillaire.

L'invention a donc pour objet un procédé de test d'une substance éventuellement active dans le domaine capillaire, caractérisé par le fait qu'on épile au moins un follicule pileux sur un sujet, que ledit follicule pileux est incubé dans un milieu de culture adéquat pendant un temps suffisant, que l'on met en contact ce follicule pileux épilé avec une substance éventuellement active dans le domaine capillaire, que l'on dose un marqueur de l'activité de ladite substance testée et que l'on évalue les résultats du dosage par rapport à un témoin.

Le follicule pileux utilisé dans le procédé selon l'invention à été isolé par épilation. L'épilation consiste en une séparation brutale du follicule pileux et du derme, généralement réalisée par une traction plus ou moins forte exercée sur la tige pilaire du follicule.
Le follicule pileux épilé peut être intact, c'est à dire comporter toutes les parties reconnues par l'homme du métier comme le constituant (voir à ce titre "Science des traitements capillaires, Charles Zviak, Masson éditeur, 1987). On citera par exemple, et de manière non limitative, parmi ces parties, la papille dermique, le bulbe pilaire, les gaines épithéliales, la glande sébacée.
Mais, bien entendu, l'invention ne se limite pas au follicule pileux épilé intact et concerne également tout follicule pileux épilé qui après isolement n'aurait conservé qu'une portion des parties le constituant.

L'épilation pour isoler le follicule pileux est un mode opératoire particulièrement intéressant puisqu'il présente les avantages d'être non invasif et donc non traumatisant pour le sujet, et d'être simple et rapide de mise en oeuvre.
Un avantage supplémentaire réside dans le fait que l'épilation peut-être pratiquée par le sujet lui-même et en n'importe quel lieu, ce qui n'ajoute pas pour le sujet de contrainte supplémentaire.

Après isolement, le follicule pileux est mis à incuber dans un milieu de culture adéquat contenant éventuellement la substance à tester. Il est bien entendu que la substance à tester peut être ajoutée au milieu d'incubation à n'importe quel moment, c'est à dire avant ou après mise en contact des cheveux épilés avec ledit milieu d'incubation. Ce milieu qui est un milieu nutritif est au moins constitué des éléments nécessaires au maintien en survie du follicule pileux.

Bien entendu, il peut contenir tout autre élément nécessaire par exemple à la croissance du follicule pileux comme de l'insuline, de la glutamine ou encore de l'hydrocortisone.

On peut citer à titre d'exemple comme milieu de culture bien connu de l'homme du métier, le milieu MEM modifié Dulbecco, le milieu Williams E, le milieu F12, le milieu de HAM, ou encore le RPMI1640, vendus par les sociétés Gibco-BRL, Biomed, Boehringer ou encore Sigma.

De manière générale, le temps d'incubation est conditionné par le temps qu'il faut au follicule pileux pour répondre à la substance éventuellement active dans le domaine capillaire avec laquelle il est mis en contact, c'est à dire au temps qu'il faut pour voir apparaître une modification du niveau d'expression du marqueur de l'activité de la substance testée que l'on dose.

Ce temps d'incubation peut aller de quelques secondes à plusieurs jours.
A titre indicatif, le temps d'incubation est généralement compris entre 5 secondes et 96 heures, et de préférence entre 12 et 24 heures.

Par marqueur de l'activité de la substance éventuellement active dans le domaine capillaire testée, on entend selon l'invention, tout élément dont la présence, l'absence, la modification de l'expression ou la modification de la distribution peuvent être mesurées, en réponse à la mise en contact du follicule pileux épilé avec ladite substance à tester. On peut citer à titre d'exemple de marqueur, et sans limitation, protéine, ADN, ARN, organite, ion, métal, acide aminé, lipide et composé liposoluble.

L'activité de la substance à tester est ainsi représentée par la variation du marqueur de l'activité de la substance testée que l'on aura choisi de doser.
Par variation, on entend toute modification de la quantité, de la concentration, de la répartition du marqueur que l'on dose.

Pour cela, le procédé selon l'invention comprend une étape de dosage du marqueur de l'activité de la substance testée.

Après incubation, ce dosage peut s'effectuer directement sur le milieu de culture pour les éléments excrétés par la cellule ou dans le follicule pileux pour les éléments non excrétés.

Ainsi, et plus particulièrement dans le cas où l'élément recherché n'est pas excrété, on peut envisager une étape supplémentaire avant le dosage, au cours de laquelle le follicule pileux est broyé, afin de rendre plus accessible le marqueur de l'activité de la substance testée à doser.

Bien évidemment, quel que soit le mode de mise en oeuvre du procédé selon l'invention, toute méthode de dosage connue par l'homme du métier peut être utilisée.
On peut, à titre d'exemple et sans limitation, citer les méthodes de dosage des protéines ou des acides nucléiques par colorimétrie, par électrophorèse, par transcriptase reverse et amplification par la technique des polymérisations en chaîne, la spectrographie de masse, la chromatographie (en phase gazeuse ou sur plaque), méthodes immunologiques, ou encore la microscopie optique ou électronique pour la mesure de la quantité d'un organite.

Le résultat du dosage, qui représente la variation du marqueur de l'activité de la substance testée que l'on a choisi de doser, n'est en soi pas exploitable directement. Il ne devient intéressant que dans la mesure où il est comparé au résultat du même dosage effectué dans les mêmes conditions, mais en l'absence de toute mise en contact du follicule pileux épilé avec la substance à tester.
C'est ainsi que le procédé selon l'invention inclut une étape au cours de laquelle on évalue les résultats du dosage par rapport à un témoin.
L'homme du métier détermine aisément, par habitude, la nature du témoin nécessaire dans la mise en oeuvre du procédé.

Un avantage de l'invention est qu'elle procure, dans le domaine du capillaire, une méthode de test d'une substance éventuellement active à la fois simple, rapide et efficace, ne faisant pas intervenir d'étapes invasives.

Dans le domaine capillaire, les substances éventuellement actives que l'on cherche à tester sont généralement en relation avec une modification de l'état de la chevelure du sujet et préférentiellement l'état présent ou futur de celle-ci.

Généralement, les substances à tester ont éventuellement une influence soit sur la couleur des follicules pileux, soit sur la densité, sur la quantité ou sur la qualité de ceux-ci. Ce sont des substances qui ont éventuellement une influence par exemple sur le ralentissement, l'arrêt de la croissance, la chute ou encore la pousse exacerbée des follicules pileux.

Ainsi la présente invention permet de tester des substances éventuellement actives dans le domaine capillaire ayant une influence sur le ralentissement, l'arrêt de la croissance, la chute, la coloration ou encore la pousse exacerbée des follicules pileux.

Préférentiellement, le procédé selon l'invention permet de tester des substances ayant éventuellement une influence sur le ralentissement, l'arrêt de la croissance, la chute ou la coloration des follicules pileux.

Si l'on s'en tient au ralentissement, à l'arrêt de la croissance ou à la chute des follicules pileux, la conséquence à terme pour le sujet est une alopécie plus ou moins prononcée, dont on sait qu'elle peut avoir des conséquences esthétiques et psychologiques et sociales pour le sujet atteint.

Plus particulièrement, le procédé selon l'invention permet de tester des substances ayant éventuellement une influence sur le ralentissement, l'arrêt de la croissance ou la chute des follicules pileux.

Le procédé selon l'invention permet donc de tester des substances éventuellement actives dans le domaine capillaire dans le traitement d'une alopécie.

Le terme alopécie recouvre toute une famille d'atteintes du follicule pileux ayant pour conséquence, quelqu'en soit la raison, la perte définitive partielle ou générale des cheveux.
On peut citer par exemple l'alopécie androgénétique, l'alopecia areata (la pelade), ou l'alopecia totalis, ou encore l'alopecia universalis.

Sans vouloir se lier à une quelconque théorie de l'invention, il semble que certaines alopécies passent par au moins une phase inflammatoire.

Une phase inflammatoire de l'alopécie se caractérise, entre autres, par une modification du niveau d'expression de médiateurs de l'inflammation.

Ainsi, l'activité d'une substance éventuellement active sur au moins une phase inflammatoire d'une alopécie peut donc être évaluée par le procédé selon l'invention en dosant dans un follicule pileux au moins un des médiateurs connus de l'inflammation, après incubation du follicule pileux avec ladite substance à tester.

Par conséquent, avantageusement, après incubation du follicule pileux avec la substance à tester, le marqueur de l'activité de ladite substance est, dans le procédé selon l'invention, un des médiateurs de l'inflammation.

Parmi ces médiateurs, on peut citer les cytokines, dont en particulier l'interleukine 1-α, l'interleukine 1-β, l'interleukine 6, les facteurs de nécrose tumorale-α et β (TNF-α et β), les chémokines comme l'interleukine 8 ou le facteur chimiotactique et activateur des monocytes (MCAF), ou encore d'autres facteurs chimiotactiques responsables du recrutement des cellules lymphocytaires, monocytaires, de Langerhans ou basophiles au niveau du site inflammatoire, tels que les leukotriènes B-4 ou encore d'autres facteurs impliqués de la cascade inflammatoire, tels que l'acide arachidonique ou les prostaglandines, dont en particulier les prostaglandines E2.

La demanderesse a en effet trouvé que chez certains sujets, et en particulier chez ceux présentant un début d'alopécie, le taux des interleukines est modifié. Le taux d'interleukine 1-α et d'interleukine 8, et plus particulièrement le taux d'interleukine 1-α, est augmenté chez la plupart des sujets présentant un début d'alopécie.

Préférentiellement, le médiateur de l'inflammation à doser dans le procédé selon l'invention est l'interleukine 1-α et l'interleukine 8, plus particulièrement le taux d'interleukine 1-α

La demanderesse a également trouvé que chez certains sujets présentant une alopécie avancée le taux des prostaglandines E2 est plus élevé que chez d'autres, suggérant une implication de ce médiateur dans la progression de ce désordre. Ainsi un dosage précoce d'une quelconque variation du taux de prostaglandines E2 permet de pronostiquer une aggravation de l'alopécie. Il est alors possible de suggérer un traitement approprié.

Ainsi, les prostaglandines E2 sont un autre médiateur de l'inflammation lié à un désordre capillaire que le procédé selon l'invention peut doser.

Un des avantages de l'invention est donc de procurer un procédé simple d'évaluation de l'activité d'une substance éventuellement active sur au moins une phase inflammatoire d'une alopécie, substance qui par la suite pourrait être utilisée dans la préparation d'une composition cosmétique ou d'un médicament en vue de traiter au moins une phase inflammatoire de l'alopécie.

Un autre avantage de l'invention est de procurer un moyen simple pour évaluer l'efficacité d'un traitement appliqué à un sujet, en mettant en oeuvre de manière répétitive et régulière à partir d'un follicule pileux épilé dudit sujet le procédé selon l'invention.

De plus, le procédé selon l'invention peut permettre d'éviter un traitement contraignant, lourd et coûteux chez un sujet particulier (dont par exemple l'alopécie n'est pas ou n'est plus en phase inflammatoire) ou lorsque le test montre une réponse à cet actif sous la forme d'une hyperproduction de médiateurs inflammatoires (phénomènes d'allergie et ou d'irritation).

De plus, le procédé selon l'invention permet d'évaluer l'activité d'une substance qui peut être utilisée pour la préparation d'une composition cosmétique ou d'un médicament en vue de traiter au moins une phase inflammatoire de l'alopécie.

A ce titre, la demanderesse a montré, comme on le verra dans les exemples ci-après, que la vitamine D et ses dérivés, et plus particulièrement la 1,25 dihydroxyvitamine D3, a une activité sur au moins un des médiateurs de l'inflammation lié à l'alopécie.

Un autre objet de l'invention est l'utilisation du procédé selon l'invention, pour évaluer l'efficacité d'un traitement appliqué à un sujet.

On va maintenant donner à titre d'illustration des exemples qui ne sauraient limiter en aucune façon la portée de l'invention.

### Exemple 1:

Effet inhibiteur de la vitamine D3 sur la production d'interleukine 1-α, induite par de l'interleukine 1-β, dans un follicule pileux épilé.

Afin de déclencher un processus mimant une phase inflammatoire dans le follicule pileux épilé, ce qui représente l'état du follicule chez un sujet en phase inflammatoire de l'alopécie, on incube au moins un follicule épilé en présence d'interleukine 1-β.

Dix cheveux épilés sont prélevés dans la zone du vertex sur un sujet volontaire.
Cinq de ces cheveux sont immédiatement mis en incubation dans du milieu William's E (vendu par la société Gibco BRL), milieu additionné (penicillin-G, 100 unités /ml ; streptomycine-S, 100 µg/ml, amphotericine 250 ng/ml) et d'interleukine 1-β (recombinante commercialisée par Saxon Biochemicals Gmbh) à raison de 100 ng/ml.
Les cinq autres cheveux sont mis en incubation dans le même milieu (additionné d'antibiotique et d'interleukine 1-β) en présence de 1,25 dihydroxyvitamine D3 (France Biochem) à la concentration de 0,1 nM.

Après 20 heures d'incubation, les surnageants de culture sont collectés dans un tube puis centrifugés 5 minutes à 14000 tours/minute (Centrifugeuse Eppendorff, modèle 5415C). Les surnageants sont alors collectés dans un tube propre et placés à 4°C. La concentration en interleukine 1-α est ensuite évaluée sur 100µl de surnageant au moyen d'un kit ELISA Biotrak commercialisé par la société Amersham, suivant les recommandations du fournisseur.

| Concentration en IL1-α (en pg/ml) | | | |
|---|---|---|---|
| | Témoin | Témoin + Vit.D3 | % Inhibition |
| Sujet n°1 | 3,8 | 2,1 | 44 |
| Sujet n°2 | 80,7 | 40,5 | 50 |
| Sujet n°3 | 13,8 | 8,8 | 36 |
| Sujet n°4 | 34,7 | 28,9 | 17 |
| Sujet n°5 | 18 | 12 | 33 |

Des analyses statistiques ont été effectuées par comparaisons des médianes par le calcul du t de Student (D.Schwartz : Méthodes statistiques à l'usage des médecins et des biologistes. Flammarion médecine et sciences 1989).

On formule l'hypothèse nulle (H0) comme étant : Témoin + Vit.D3 ≥ Témoin, dans le cadre d'un test unilatéral, tenant compte du fait qu'il s'agit de valeurs appariées.

Selon Student pour le t calculé, il y a rejet de H0 au seuil de p = 5%. Ici p = 0,001. Conclusion: puisque H0 est rejetée, on peut conclure au seuil de 5% que les deux populations, (1 : Témoin et 2 : Témoin + Vit.D3), différent de façon significative, les résultats obtenus pour la population 1 sont significativement supérieurs à ceux obtenus avec population 2.

L'expérience montre que la vitamine D3 est un bon inhibiteur de la production d'interleukine 1-α, dans un système reproduisant les conditions inflammatoires d'un follicule pileux.
Par conséquent ce test peut être utilisé pour évaluer les capacités anti-inflammatoires d'autres analogues de la vitamine D3, par l'évaluation de leur capacité à inhiber la production d'interleukine-1-α.

### Exemple 2 :

Effet inhibiteur de la vitamine D3 sur la production d'interleukine 8, induite par de l'interleukine 1-α, dans un follicule pileux épilé.

Quinze cheveux provenant de sujets volontaires sont prélevés dans la zone de la nuque. Ils sont immédiatement incubés dans du milieu William's E commercialisé par la société Gibco BRL additionné de glutamine (2 mM) et d'antibiotiques (penicillin-G, 100 unités/ml; streptomycine-S, 100 µg/ml, amphotérine 250 ng/ml) à raison de 200 µl de milieu par cheveu épilé.

Ils sont ensuite divisés en trois lots:

Lot n°1 : Cheveux incubés dans le milieu William's E mentionné plus haut.

Lot n°2 : Cheveux incubés dans le milieu William's E mentionné plus haut, additionné d'interleukine 1-α (Biosource International) à une concentration finale de 25 ou 100 ng/ml (25 ng/ml pour le sujet n°1 ou 100 ng/ml pour les sujets n°2 et 3).

Lot n°3 : Cheveux incubés dans le même milieu que le lot n°2 additionné de 1-25 dihydroxyvitamine D3 à 0.1 nM.

Au bout de 20 heures, les surnageants de culture sont collectés dans un tube puis centrifugés 5 minutes à 14000 tours/minutes (Centrifugeuse Eppendorff, modèle 5415C). Les surnageants sont ensuite collectés dans un tube propre et placés à 4°C.

La concentration d'interleukine 8 est ensuite évaluée sur 100 µl de surnageant au moyen d'un kit ELISA Biotrak commercialisé par la société Amersham en suivant les instructions du fabriquant.

| | Lot n°1 | Lot n°2 | Lot n°3 | |
|---|---|---|---|---|
| | IL-8 (en pg/ml) | | | % Inhibition |
| Sujet n°1 | 170 | 622 | 251 | 82 |
| Sujet n°2 | 300 | 863 | 353 | 91 |
| Sujet n°3 | 266 | 657 | 496 | 41 |
| Sujet n°4 | 226 | 477 | 356 | 48 |

Des analyses statistiques ont été effectuées par comparaisons des moyennes ou des médianes par l'application du test t de Student (D.Schwartz/Méthodes statistiques à l'usage des médecins et des biologistes. Flammarion médecine et sciences 1989).
On formule l'hypothèse nulle (H0) comme étant : Lot n°3 ≥ Lot n°2, dans le cadre d'un test unilatéral, tenant compte du fait qu'il s'agit de valeurs appariées.

Selon Student pour le t calculé, il y a rejet de H0 au seuil de p = 5%. Ici p = 0,025. Conclusion: puisque H0 est rejetée, on peut conclure au seuil de 5% que les deux lots différent de façon significative, les résultats obtenus pour le lot 2 sont significativement supérieurs à ceux obtenus avec le lot 3.

Dans ces 4 cas, on observe une inhibition de la production d'interleukine 8 par la 1-25 dihydroxyvitamine D3.

L'IL-8 étant une chemokine inflammatoire induite par l'interleukine 1-α, l'essai montre que la 1-25 dihydroxyvitamine D3, à 0.1 nM a un effet anti-inflammatoire sur le follicule épilé, incubé en présence d'interleukine 1-α.

Par conséquent ce test peut être utilisé pour évaluer les capacités anti-inflammatoires d'autres analogues de la vitamine D3, par l'évaluation de leur capacité à inhiber la production d'interleukine-8.

Exemple 3: Effet inhibiteur du minoxidil sur la production de prostaglandines E2 (PGE2), dans un follicule pileux isolé par épilation.

10 cheveux provenant de trois donneurs alopéciques et de deux donneurs non-alopéciques sont prélevés par épilation, dans la zone du vertex. Ils sont répartis immédiatement en deux lots:

Lot n°1 : 5 cheveux incubés dans du milieu William's E (commercialisé par la société Gibco BRL) additionné de glutamine (2 mM) et d'antibiotiques (penicillin-G, 100 unités /ml; streptomycine-S, 100 µg/ml, amphotericine 250 ng/ml).
Lot n°2 : 5 cheveux incubés dans le même milieu que précédemment mais contenant en plus du minoxidil à la concentration finale de 10 µM.

Au bout de 18 heures, les épilats de chaque groupe (5 cheveux) sont collectés dans un microtube sous une atmosphère d'argon puis stockés à -80°C. Le jour du dosage, on rajoute 250 µl de méthanol dégazé dans chaque tube puis chaque échantillon est broyé mécaniquement (10 rotations) à l'aide d'un pilon (tissue grind pestle SZ 20, commercialisé par la société Kontes). Le broyat est ensuite soniqué (20 impulsions d'une seconde ; 50% d'amplitude) à l'aide d'un sonicateur "Vibra cell 72 434" (commercialisé par la société Bioblock Scientific). Le broyat soniqué est centrifugé à 4°C à 14000 tours/minutes pendant 10 minutes (Centrifugeuse Eppendorff, modèle 5415C). Le surnageant est ensuite collecté dans un tube propre et lyophilisé pendant une heure. Le lyophilisat est repris dans 60 µl de tampon phosphate pH 7.5 fourni dans le kit Biotrak (commercialisé par la société Amersham). Le contenu en PGE2 de 50 µl de cette préparation est ensuite évalué au moyen du KIT Biotrak selon les indications du fabriquant.

| | Lot n°1 | Lot n°2 | |
|---|---|---|---|
| | PGE2 en pg/ml | | % Inhibition |
| Donneur #1 (alopécique) | 15.2 | 5.6 | 63 |
| Donneur #2 (alopécique) | 13.7 | 8.5 | 38 |
| Donneur #3 (alopécique) | 15.7 | 15.3 | 2,5 |
| Donneur #4 (non alopécique) | 6,2 | 6,2 | 0 |
| Donneur #5 (non alopécique) | 10,7 | 11,2 | 0 |

Ces exemples montrent qu' in vitro, on est capable, au moyen d'un agent pharmacologique réputé comme favorisant la repousse du cheveu in vivo (i.e. le Minoxidil), de diminuer la quantité de PGE2 produite par les épilats de certains individus alopéciques en phase inflammatoire (donneurs 1 et 2 dans cet exemple). Cette observation peut orienter un traitement au minoxidil in vivo chez ces individus qui répondent au minoxidil in vitro et de façon générale, cette méthodologie peut orienter le choix d'un actif agissant, sur le critère étudié (ici, la production de PGE2) pour un donneur particulier.
Inversement cette méthode d'évaluation permet d'éviter un traitement contraignant, lourd et coûteux chez un sujet particulier (exemple du donneur 3) ou lorsque le test montre une réponse à cet actif sous la forme d'une hyperproduction de médiateurs inflammatoires (phénomènes d'allergie et ou d'irritation).

## Revendications

1. Procédé de test d'une substance éventuellement active dans le domaine capillaire, caractérisé par le fait qu'on épile au moins un follicule pileux sur un sujet, que ledit follicule pileux épilé est incubé dans un milieu de culture adéquat pendant un temps suffisant, que l'on met en contact ce follicule pileux épilé avec une substance éventuellement active dans le domaine capillaire, que l'on dose un marqueur de l'activité de ladite substance testée et que l'on évalue les résultats du dosage par rapport à un témoin.

2. Procédé selon la revendication précédente, caractérisé par le fait que le milieu de culture comprend au moins un élément nécessaire au maintien en survie du follicule pileux.

3. Procédé selon l'une quelconque des revendications 1 ou 2, caractérisé par le fait que le milieu de culture comprend au moins un élément nécessaire à la croissance du follicule pileux.

4. Procédé selon l'une quelconque des revendications précédentes, caractérisé par le fait que le temps d'incubation est compris entre 5 secondes et 96 heures.

5. Procédé selon la revendication précédente, caractérisé par le fait que le temps d'incubation est compris entre 12 et 24 heures.

6. Procédé selon l'une quelconque des revendications précédentes, caractérisé par le fait que l'on dose le marqueur de l'activité de ladite substance testée directement dans le milieu de culture.

7. Procédé selon l'une quelconque des revendications 1 à 5, caractérisé par le fait que l'on dose le marqueur de l'activité de ladite substance testée dans le follicule pileux.

8. Procédé selon la revendication précédente, caractérisé par le fait qu'avant le dosage du marqueur de l'activité de ladite substance testée, on procède au broyage du follicule pileux.

9. Procédé selon l'une quelconque des revendications précédentes, caractérisé par le fait que le marqueur de l'activité de ladite substance testée est un acide nucléique, une protéine ou un groupe de protéines liées ou non, un ion, un organite cellulaire, un lipide, ou un polyoside.

10. Procédé selon l'une quelconque des revendications précédentes, caractérisé par le fait que l'on teste une substance éventuellement active dans le domaine capillaire ayant une influence sur le ralentissement, l'arrêt de la croissance, la chute, la coloration ou encore la pousse exacerbée des follicules pileux.

11. Procédé selon la revendication précédente, caractérisé par le fait que l'on teste une substance ayant éventuellement une influence sur le ralentissement, l'arrêt de la croissance, la chute ou la coloration des follicules pileux.

12. Procédé selon la revendication précédente, caractérisé par le fait que l'on teste une substance ayant éventuellement une influence sur le ralentissement, l'arrêt de la croissance ou la chute des follicules pileux.

13. Procédé selon la revendication précédente, caractérisé par le fait que l'on teste une substance ayant éventuellement une influence dans le traitement de l'alopécie.

14. Procédé selon l'une quelconque des revendications précédentes, caractérisé par le fait que l'on dose au moins un des médiateurs de l'inflammation en tant que marqueur de l'activité de ladite substance testée.

15. Procédé selon la revendication précédente, caractérisé par le fait que l'on dose au moins un médiateur choisi parmi les cytokines, dont en particulier l'interleukine 1-α, l'interleukine 1-β, l'interleukine 6, les facteurs de nécrose tumorale-α et β (TNF-α et β), les chémokines comme l'interleukine 8 ou le facteur chimiotactique et activateur des monocytes (MCAF), ou encore d'autres facteurs chimiotactiques responsables du recrutement des cellules lymphocytaires, monocytaires, de Langerhans ou basophiles au niveau du site inflammatoire, tels que les leukotriènes B-4 ou encore d'autres facteurs impliqués de la cascade inflammatoire, tels que l'acide arachidonique ou les prostaglandines, dont en particulier les prostaglandines E2.

16. Procédé selon la revendication précédente, caractérisé par le fait que l'on dose au moins un médiateur choisi parmi les interleukines.

17. Procédé selon la revendication précédente, caractérisé par le fait que l'on dose l'interleukine 1-α.

18. Procédé selon la revendication 16, caractérisé par le fait que l'on dose l'interleukine 8.

19. Procédé selon la revendication 15, caractérisé par le fait que l'on dose les prostaglandines.

20. Procédé selon la revendication précédente, caractérisé par le fait que l'on dose les prostaglandines E2.

21. Procédé selon l'une quelconque des revendications précédentes, caractérisé par le fait que l'on teste une substance ayant éventuellement une activité sur au moins une phase inflammatoire de l'alopécie.

22. Procédé selon l'une quelconque des revendications précédentes, caractérisé par le fait que l'on teste une substance éventuellement active dans le domaine capillaire permettant de diminuer, voire d'abolir, au moins une phase inflammatoire de l'alopécie.

23. Utilisation du procédé selon l'une quelconque des revendications 1 à 22, pour évaluer l'efficacité d'un traitement appliqué à un sujet.

## Patentansprüche

1. Verfahren zum Testen einer Substanz, die möglicherweise im Haarbereich wirksam ist,
**dadurch gekennzeichnet,** daß einer Versuchsperson mindestens ein Haarfollikel durch Herausziehen entnommen wird, das herausgezogene Haarfollikel während einer ausreichenden Zeit in einem geeigneten Kulturmedium inkubiert wird, das entnommene Haarfollikel mit einer möglicherweise im Haarbereich wirksamen Substanz in Kontakt gebracht wird, ein Marker für die Aktivität dieser getesteten Substanz bestimmt wird und die Ergebnisse der Bestimmung in Bezug auf eine Kontrolle bewertet werden.

2. Verfahren nach dem vorhergehenden Anspruch, dadurch gekennzeichnet, daß das Kulturmedium mindestens ein Element enthält, das notwendig ist, um das Haarfollikel am Leben zu erhalten.

3. Verfahren nach Anspruch 1 oder 2, dadurch gekennzeichnet, daß das Kulturmedium mindestens ein Element enthält, das zum Wachstum des Haarfollikels notwendig ist.

4. Verfahren nach einem der vorhergehenden Ansprüche, dadurch gekennzeichnet, daß die Inkubationszeit im Bereich von 5 Sekunden bis 96 Stunden liegt.

5. Verfahren nach dem vorhergehenden Anspruch, dadurch gekennzeichnet, daß die Inkubationszeit im Bereich von 12 bis 24 Stunden liegt.

6. Verfahren nach einem der vorhergehenden Ansprüche, dadurch gekennzeichnet, daß der Marker für die Aktivität der getesteten Substanz direkt im Kulturmedium bestimmt wird.

7. Verfahren nach einem der Ansprüche 1 bis 5, dadurch gekennzeichnet, daß der Marker für die Aktivität der genannten getesteten Substanz im Haarfollikel bestimmt wird.

8. Verfahren nach dem vorhergehenden Anspruch, dadurch gekennzeichnet, daß vor der Bestimmung des Markers für die Aktivität der getesteten Substanz eine Zerkleinerung des Haarfollikels durchgeführt wird.

9. Verfahren nach einem der vorhergehenden Ansprüche, dadurch gekennzeichnet, daß der Marker für die Aktivität der getesteten Substanz eine Nucleinsäure, ein Protein oder eine Gruppe von gebundenen oder nicht gebundenen Proteinen, ein Ion, eine Zellorganelle, ein Lipid oder ein Polysaccharid ist.

10. Verfahren nach einem der vorhergehenden Ansprüche, dadurch gekennzeichnet, daß eine möglicherweise im Haarbereich wirksame Substanz getestet wird, die einen Einfluß auf die Verlangsamung des Wachstums, den Stillstand des Wachstums, den Ausfall, die Färbung oder auch das übermäßige Wachstum von Haarfollikeln aufweist.

11. Verfahren nach dem vorhergehenden Anspruch, dadurch gekennzeichnet, daß eine Substanz getestet wird, die möglicherweise einen Einfluß auf die Verlangsamung des Wachstums, den Stillstand des Wachstums, den Ausfall oder die Färbung von Haarfollikeln aufweist.

12. Verfahren nach dem vorhergehenden Anspruch, dadurch gekennzeichnet, daß eine Substanz getestet wird, die möglicherweise einen Einfluß auf die Verlangsamung des Wachstums, den Stillstand des Wachstums oder den Ausfall von Haarfollikeln aufweist.

13. Verfahren nach dem vorhergehenden Anspruch, dadurch gekennzeichnet, daß eine Substanz getestet wird, die möglicherweise einen Einfluß bei der Behandlung der Alopezie aufweist.

14. Verfahren nach einem der vorhergehenden Ansprüche, dadurch gekennzeichnet, daß mindestens ein Entzündungsmediator als Marker für die Aktivität der getesteten Substanz bestimmt wird.

15. Verfahren nach dem vorhergehenden Anspruch, dadurch gekennzeichnet, daß mindestens ein Mediator bestimmt wird, der ausgewählt ist unter den Cytokinen, insbesondere Interleukin 1α, Interleukin 1β, Interleukin 6, den Tumornekrose-Faktoren α und β (TNF-α und TNF-β), Chemokinen, wie Interleukin 8 oder dem chemotaktischen aktivierenden Faktor der Monozyten (MCAF), anderen chemotaktischen Faktoren, die für die Lieferung von Lymphozyten, Monozyten, Langerhans-Zellen oder basophilen Zellen im Bereich einer Enzündungsstelle verantwortlich sind, wie den Leukotrienen B-4, oder anderen Faktoren, die an der Entzündungskaskade beteiligt sind, wie Arachidonsäure oder Prostaglandinen, insbesondere den Prostaglandinen E2.

16. Verfahren nach dem vorhergehenden Anspruch, dadurch gekennzeichnet, daß mindestens ein Mediator, der unter den Interleukinen ausgewählt ist, bestimmt wird.

17. Verfahren nach dem vorhergehenden Anspruch, dadurch gekennzeichnet, daß das Interleukin 1-α bestimmt wird.

18. Verfahren nach Anspruch 16, dadurch gekennzeichnet, daß Interleukin 8 bestimmt wird.

19. Verfahren nach Anspruch 15, dadurch gekennzeichnet, daß Prostaglandine bestimmt werden.

20. Verfahren nach dem vorhergehenden Anspruch, dadurch gekennzeichnet, daß die Prostaglandine E2 bestimmt werden.

21. Verfahren nach einem der vorhergehenden Ansprüche, dadurch gekennzeichnet, daß eine Substanz getestet wird, die möglicherweise eine Wirksamkeit auf mindestens eine entzündliche Phase der Alopezie besitzt.

22. Verfahren nach einem der vorhergehenden Ansprüche, dadurch gekennzeichnet, daß eine im Haarbereich möglicherweise wirksame Substanz getestet wird, durch die sich zumindest eine entzündliche Phase der Alopezie verringern oder ausschalten läßt.

23. Anwendung des Verfahrens nach einem der Ansprüche 1 bis 22 zur Bewertung der Wirksamkeit einer Behandlung, die bei einer Versuchsperson angewandt wird.

## Claims

1. Process for testing a substance which is possibly active in the hair field, characterized in that at least one hair follicle is plucked from a subject, in that the said plucked hair follicle is incubated in a suitable culture medium for a sufficient time, in that this plucked hair follicle is brought into contact with a substance which is possibly active in the hair field, in that a label of the activity of the said tested substance is quantitatively determined and in that the results of the quantitative determination are evaluated with respect to a control.

2. Process according to the preceding claim, characterized in that the culture medium comprises at least one component necessary for keeping the hair follicle alive.

3. Process according to either of Claims 1 and 2, characterized in that the culture medium comprises at least one component necessary for the growth of the hair follicle.

4. Process according to any one of the preceding claims, characterized in that the incubation time is between 5 seconds and 96 hours.

5. Process according to the preceding claim, characterized in that the incubation time is between 12 and 24 hours.

6. Process according to any one of the preceding claims, characterized in that the label of the activity of the said tested substance is quantitatively determined directly in the culture medium.

7. Process according to any one of Claims 1 to 5, characterized in that the label of the activity of the said tested substance is quantitatively determined in the hair follicle.

8. Process according to the preceding claim, characterized in that, before the quantitative determination of the label of the activity of the said tested substance, the hair follicle is ground.

9. Process according to any one of the preceding claims, characterized in that the label of the activity of the said tested substance is a nucleic acid, a protein or a group of proteins, which may or may not be bound, an ion, a cell organelle, a lipid or a polysaccharide.

10. Process according to any one of the preceding claims, characterized in that a substance which is possibly active in the hair field which has an effect on the slowing down or halting of the growth, the loss, the colouring or alternatively the intensified growth of the hair follicles is tested.

11. Process according to the preceding claim, characterized in that a substance possibly having an effect on the slowing down or halting of the growth, the loss or the colouring of the hair follicles is tested.

12. Process according to the preceding claim, characterized in that a substance possibly having an effect on the slowing down or halting of the growth or the loss of the hair follicles is tested.

13. Process according to the preceding claim, characterized in that a substance possibly having an effect on the treatment of alopecia is tested.

14. Process according to any one of the preceding claims, characterized in that at least one of the mediators of inflammation is quantitatively determined as label of the activity of the said tested substance.

15. Process according to the preceding claim, characterized in that at least one mediator chosen from cytokines, including in particular interleukin-1α, interleukin-1β, interleukin-6 or tumour necrosis factors α and β (TNF-α and -β), chemokines, such as interleukin-8 or monocyte chemotactic and activating factor (MCAF), or alternatively other chemotactic factors responsible for the recruitment of lymphocyte, monocyte, Langerhans or basophil cells at the inflammatory site, such as leukotrienes B₄, or alternatively other factors involved in the inflammatory cascade, such as arachidonic acid or prostaglandins, including in particular prostaglandins E₂, is quantitatively determined.

16. Process according to the preceding claim, characterized in that at least one mediator chosen from interleukins is quantitatively determined.

17. Process according to the preceding claim, characterized in that interleukin-1α is quantitatively determined.

18. Process according to Claim 16, characterized in that interleukin-8 is quantitatively determined.

19. Process according to Claim 15, characterized in that prostaglandins are quantitatively determined.

20. Process according to the preceding claim, characterized in that prostaglandins E₂ are quantitatively determined.

21. Process according to any one of the preceding claims, characterized in that a substance possibly having an activity against at least one inflammatory stage of alopecia is tested.

22. Process according to any one of the preceding claims, characterized in that a substance which is possibly active in the hair field, making it possible to decrease, indeed eliminate, at least one inflammatory stage of alopecia, is tested.

23. Use of the process according to any one of Claims 1 to 22 for evaluating the effectiveness of a treatment applied to a subject.
